# EUROPEAN PATENT APPLICATION

(11) **EP 2 937 082 A1**
(43) Date of publication of application: **28.10.2015**
(21) Application number: 14190253.6
(22) Date of filing: 24.10.2014
(51) Int. Cl.: A61K 9/48, A61K 47/38, A61K 8/00

(54) **Film-forming composite used to make starch softgel capsules and the method to prepare it**

(30) Priority: 21.04.2014 CN 201410159754
(71) Applicant: Hunan Er-Kang Pharmaceutical Co., Ltd., Changsha Hunan 410331 (CN)
(72) Inventor: Shuai, Fangwen, 410331 Changsha (CN); Wang, Xiangfeng, 410331 Changsha (CN); Zhang, Jiawei, 410331 Changsha (CN)
(74) Representative: Distefano, Luigi Roberto

(57) **Abstract**

This present invention relates to a film-forming composite used to make starch softgel capsules and the method to prepare it. The said composite is made from water and hydroxypropyl starch by specific percentage, plus (optional) light-screening agent, colorant, and flavoring agent. Mix up the above components in sequential order and then process the mix into film-forming composite through controlled heating. The composite such made can be directly used to make starch softgel capsules by the conventional method applied for the gelatin capsule production.

## Description

### CROSS REFERENCE TO RELATED APPLICATION

This application claims priority from Chinese Patent Application No. 201410159754.1, filed with State Intellectual Property Office, P. R. C. on April 21, 2014, the entire content of which is incorporated herein by reference.

### FIELD OF INVENTION

This present invention relates to a composite used to make starch softgel capsules and the method to prepare it, which falls into biomedicine industry.

### BACKGROUND OF THE INVENTION

Capsule dosage, as one of the most popular pharmaceutical dosage forms, is widely favored by pharmaceutical manufacturers and research institutes due to its short R&D cycle and relatively easy preparation process.

The raw material of traditional capsules is gelatin, a type of non-bioactive collagen hydrolysate extracted from animal skins or bones, swelling in cold water and dissolved into gel solution in hot water. For a quite long period of time, gelatin capsules have almost dominated the whole capsule market, and even today, they still occupy over 90% market share. At present, there is a huge demand for capsules in the whole country, which is estimated to be hundreds of billions of capsules consumed each year. However, the source of gelatin is so limited! Normally, the industrial gelatin is made of recycled waste leather and the leftover from leather processing. The gelatin made this way is strictly prohibited from being used as edible or pharmaceutical ingredients, and the animal skins and bones from legitimate sources are so limited that they are a far cry from meeting the market demands! Driven by huge profit, some manufacturers use illegal industrial gelatin to produce capsules, which impose serious threat to human health.

Starch is processed from natural plant source and the processing method is simple. The hydroxypropyl starch obtained by propylene oxide etherification, when mixed with water by certain proportion and at proper temperature, can be gelatinized to gel solution that meets the technical requirements for capsule production.

The research on capsule manufacturing technology using starch as the raw material has been conducted by experts and scientists in the industry. For example, the Patent CN 100393296C disclosed a composite used to produce capsules and the method to prepare it. This composite, composed of water, starch, carrageenan, surface active agent, and plasticizer, is not limited to certain group of applicable Users; and its cost is significantly lower than that of the capsules made from starch derivatives. Another example is Patent CN 103191433, which disclosed a hollow capsule made from starch and its preparation method. The technical formula is as follows: purified water (81-87%), plant starch (12.5-17.5%), carrageenan (0.5-1%), glycerol (0.5-0.7%), sodium dodecyl sulfate (0.03-0.05%), and medicinal colorant (0.1-0.2%). The advantage of this technical proposal lie in that, no gelatin is used as raw material, and thus avoids excessive heavy metal chromium which usually exists in gelatin capsules.

Patent CN103228269A disclosed a film-forming composite used to produce softgel capsule, which consists of waxy maize starch decomposed by acid, gelling agent; and plasticizer. Additionally, the capsule shell prepared using the film-forming composite disclosed in this patent has superior characteristics in physical strength, disintegrating ability, smell, color, and stability.

CN103495176A disclosed a preparation method of starch-based capsules, wherein, the composite starch base and the gel are mixed and extruded into a composite starch film, and then this film is processed into starch-based film by rotary die method. CN103550065A disclosed a preparation method of starch-based capsules, in which, premixed starch and gelling agent are extruded into a film respectively, and the two films are then compounded into a composite starch film by a multi-layer co-extrusion machine. This composite film is processed into softgel capsules by rotary die method. The existing technologies described in these two patents both disclosed the film-forming composite used to produce said softgel capsules

However, there are still some defects such as unsatisfactory friability and overlong disintegration time, and the encapsulation performance is also not good enough to meet the pharmacopeial requirement.

### SUMMARY OF THE INVENTION

An embodiment of the invention provides a starch composite used to make starch softgel capsules and the method to prepare it, as well as the softgel capsules made from this starch composite. The softgel capsules such made have the advantages of good encapsulation performance, broad adjustable range in softness, viscosity, and disintegrating property, non-animal sourced ingredients, and low content of heavy metal impurities.

First, the invention discloses a composite used to prepare starch capsules, which contains the following components:

Hydroxypropyl starch (20∼95%), appropriate adjuvant used to improve softgel capsule performance (0∼25%), water (5∼75%), light screening agent (0∼2%), Colorant: (0-2%), flavoring agent (0∼2%). The percentages of the above components sum up to 100%.

The said hydroxypropyl starch can be obtained by hydroxypropylating the starches processed from natural plants like cassava, corn, pea, potato, wheat, rice, etc, and the hydroxypropyl content in the said hydroxypropyl starch is 2.0∼7.0%, with 3.2∼5.0% being the most optimal range.

The adjuvant used to improve softgel capsule performance is one or a combination of the substances selected from the group consisting of gelling agent, water retention agent, film-forming agent by the percentage of gelling agent 0∼55%, softening agent 0∼65%, and film-forming agent 0∼5%; The said gelling agent is natural plant polysaccharides or microbial extracellular polysaccharide, with the most optimal choice being one or a combination of the substances from the group consisting of carrageenan, xanthan gum, guar gum, seaweed polysaccharides, pectin, hydroxypropyl methyl cellulose, and carboxymethyl cellulose;

the softening agent is multiple fatty alcohol or Aliphatic polyol polymer, with the most optimal choice being one or a combination of the substances selected from the group consisting of glycerin, polyethylene glycol 200, and polyethylene glycol 400; the film-forming agent is selected from soluble calcium salt and potassium salt, with the most optimal choice being one or a combination of the chemicals from the group consisting of calcium chloride, potassium chloride, calcium citrate, and potassium citrate.

The above components function to adjust the viscosity, disintegrating property, and film-forming properties of the softgel capsules made from this composite and the flexibility and toughness of the materialas well.

The said light screening agent is edetate calcium, or titanium dioxide or vanillin; the colorant is of food or pharmaceutical grade; the flavoring agent is edible essence selected from the group consisting of the essence of different flavors such as apple, mint, lemon, banana and pineapple; the light screening agent is used to avoid capsule content deterioration caused by light and other reasons. For example, vitamin A and E tend to deteriorate when exposed to light. The flavoring agent is used to mask the special ordour from certain medicines, such as the gamey smell from cod liver oil. One point needs be addressed is that these three components are not a must in the composite but are selectively added depending on the nature of the capsule nature and customers' demands.

Additionally, the invention also provides a preparation method of a composite used to prepare starch softgel capsules, which is implemented according to the following technical proposal.

### 1. Raw material prescription

| | |
|---|---|
| Hydroxypropyl starch: | 5 parts |
| Water: | 5-40 parts |

Appropriate adjuvant used to improve softgel capsule performance:

| | |
|---|---|
| | 0-2 parts, |
| Light screening agent: | 0-0.3 parts |
| Coloring agent: | 0-0.5 parts, |
| Flavoring agent: | 0-0.3 parts |

The said hydroxypropyl starch is obtained by hydroxypropylating the starches extracted from natural plants like cassava, corn, pea, potato, wheat, rice, etc. The hydroxypropyl content in the hydroxypropyl starch is 2.0∼7.0%.

The light screening agent is calcium edetate, or titanium dioxide or vanillin.

The colorant is of food or pharmaceutical grade

The flavoring agent is edible essence selected from the group consisting of the essence of different flavors such as apple, mint, lemon,banana and pineapple.

The adjuvant used to improve softgel capsule performance is one or a combination of the substances selected from the group consisting of gelling agent, water retention agent, film-forming agent by the percentage of gelling agent 0∼55%, softening agent 0∼65%, and film-forming agent 0∼5%

Further, the gelling agent is made of natural plant polysaccharides or microbial extracellular polysaccharide.

Further, the softening agent is multiple fatty alcohol or aliphatic polyol polymer.

Further, the film-forming agent is selected from soluble calcium salt and potassium salt.

Further, the gelling agent is made of natural plant polysaccharides or microbial extracellular polysaccharide, with the optimal choice being one or a combination of the substances selected from the group consisting of carrageenan, xanthan gum, guar gum, seaweed polysaccharides, pectin, hydroxypropyl methyl cellulose, carboxymethyl cellulose;

Further, the softening agent is multiple fatty alcohol or aliphatic polyol polymer, with the optimal choice being one or a combination of the substances selected from the group consisting of glycerol, polyethylene glycol 200, and polyethylene glycol 400.

Further, the film-forming agent is selected from soluble calcium salt or potassium salt, with the optimal choice being one or a combination of the chemicals selected from the group consisting of calcium chloride, potassium chloride, calcium citrate, and potassium citrate.

### 2 Technical processes:

Step 1: Preparation liquid A: Allow hydroxypropyl starch to be soaked in water in the gel tank for 0.5∼2 hours at 20-25°C to form liquid A.
Step 2: Preparation liquid B: Mix the adjuvant (used to improve softgel capsule performance), light-screening agent, and colorant in water, stir for 5-10minuts, and then heat up to 55-65°C within 10 minutes to form liquid B
Step 3: Preparation liquid C: Mix water and flavoring agent for 5-10 minutes at the temperature of 15-20 °C to form liquid C.
Step 4 : Heat up the liquid A to 80-100 °C, during which, liquid B can be added in as an option. Mix well and temperature-preservation stir for 1-2 hours to form feed-liquid D
Step 5 : Add liquid C (optional) into liquid A or Liquid D when the temperature falls to 50-60 °C. Stir and uniformly mix, maintain the temperature for 0.5∼1.5 hours. Keep airtight and save for use.

Special attention: In the above Step D, 20-35 minute temperature-preservation stirring is required at the point of 55-65 °C in the middle of heating up to 80-100 °C; If solution B is needed, then the temperature-preserved solution B should be added into Solution A at this time. Stir and well mix.

In the study on starch capsule preparation method using starch or starch derivatives, it was accidently found that if the temperature is maintained at certain rage for a period of time in the middle of the process of heating up the hydroxypropyl starch dissolved in water to 80-100 °C, the capsules prepared will present much better performance in encapsulation, disintegrating property, and anti-impact capability. Another finding is that the capsules made from the composite material prepared this way can meet the pharmacopoeial requirements in all technical parameters even with adding little or no additives of gelling agent, softener or film-forming agent.

Based on the above findings, the feed-liquid B and C are not a must, but just an optional choice in the Step (D) and (E) . No negative effects will be imposed on the capsule quality regardless of whether liquid B or C is added in the preparation process, that is to say, even if without adding liquid B or C, or any other similar functional materials, the composite formed in the preparation process as well as the capsules made from this composite can meet the quality and technical standards regulated in "Chinese Pharmacopoeia". Whether or not Liquid B or C should be added depends on the users' requirements on the performance of the final products.

In addition, the invention provides a softgel capsule made from the starch composite described above, and this capsule is directly made by dropping-pill or rotary die method.

The benefits of this invention and the technical problems solved.

(1) This invention provides a composite material that can be used to produce starch softgel capsules. This composite contains no animal derived ingredients, especially no gelatin; and its technical performance can be easily custom-tailored according to specific technical requirements, especially the performance of viscosity, gel strength, capsule disintegration properties, and impact resistance, which can be adjusted in a relatively large range within the legal scope. This solved the problems of that the existing starch capsule material cannot easily meet pharmacopoeial requirements and that limited materials cannot adapt to customers' changing demands.

This invention discloses a method to prepare a film-forming composite that can be used to produce starch softgel capsules. This method provides a solution to the problem that the existing starch-filming composite and the like need a lot of other additives to improve the gelling performance, which makes the composition of the softgel capsule shell as simple as possible, and thus reduces the possible interaction or other compatibility issues.

In addition, this film-forming can be directly processed into starch softgel capsules on existing capsule machine with no equipment upgrade or improvement needed, which reduces the burden on the manufacturers and makes it easy to popularize this preparation method.

To further illustrate the practice of this invention, the following embodiments and examples are presented. It will be understood the examples are given for illustration purpose only and not by way of limitation.

### EMBODIMENTS

Now point to the following several specific implementation example and contrast example to further this invention. It is important to note that the following example is only used to help understand the invention; further limit of this invention is not the solution.

### Embodiment 1

### Formula:

Hydroxypropyl wheat starch: 5 parts, with 3.8% hydroxypropyl content.
Water: 40 parts

Preparation steps: Soak the hydroxypropyl wheat starch in water in the gel tank at the temperature of 23 °C for 1 hours to from Liquid A. Heat up liquid A, and when the temperature reaches 60 °C, temperature-preservation stir for 25∼30 minutes, and then continue to heat up to 90 °C, staying at this temperature for 1. hour. Cool down, and when the temperature drops to 55 °C, maintain this temperature for 1 hour, staying static to defoam. Apply such made film-forming composite to make 1000 capsules by rotating die method.

### Embodiment 2

### Formula:

Hydroxypropyl corn starch: 5 parts, with 2.4% hydroxypropyl content.
Water: 20 parts

Soak the Hydroxypropyl corn starch in water in the gel tank at the temperature of 25°C for 2 hours to from Liquid A. Heat up liquid A, and when the temperature reaches 65°C, temperature-preservation stir for 35minutes, and then continue to heat up to 95 °C, staying at this temperature for 1.5 hour. Cool down, and when the temperature drops to 60°C, maintain this temperature for 1 hour. Stay static to defoam and then apply such made film-forming composite to make 1000 capsules by rotating die method.

### Embodiment 3

### Formula:

Hydroxypropyl tapioca starch: 5 parts; 5.4%. Hydroxypropyl content.
Water: 40 parts
Vanillin: 0.15 parts

### Pharmaceutical grade citric yellow: 0.06 parts

Soak the Hydroxypropyl tapioca starch in water in the gel tank at the temperature of 25 °C for 1 hours to from Liquid A. Heat up liquid A, and when the temperature reaches 60 °C, temperature-preservation stir for 30 minutes, and then continue to heat up to 85 °C, staying at this temperature for 1.5 hour. Cool down, and when the temperature drops to 50 °C, maintain this temperature for 1 hour. Stay static to defoam and then apply such made film-forming composite to make 1000 capsules by rotating die method.

### Embodiment 4

Hydroxypropyl tapioca starch: 5 parts; hydroxypropyl content: 6.1%.
Water: 40parts

Appropriate adjuvant used to improve softgel capsule performance: 1.4 parts, which include 0.7 parts carrageenan and 0.7 parts glycerol.

Soak the Hydroxypropyl tapioca starch in water in the gel tank at the temperature of 25 °C for 1.5 hours to from Liquid A. Mix carrageenan and glycerol in proper amount of water and stir for 10 minutes at 25 °C, and then heat the mix up to 60 °C to form liquid B. Heat up liquid A, and when the temperature reaches 60 °C, add liquid B into it and stir for 30 minutes, and then continue to heat up to 95 °C, staying at this temperature for 1.5 hours. Cool down, and when the temperature drops to 50 °C, add liquid C and well mix, and maintain this temperature for 1 hour. Stay static to defoam and then apply such made film-forming composite to make 1000 capsules by rotating die method.

### Embodiment 5

Hydroxypropyl pea starch: 5 parts; with 6.8% hydroxypropyl content.
Water: 10 parts

Appropriate adjuvant used to improve softgel capsule performance: 2 parts, which include 0.5 parts carrageenan, 0.2 parts pectin, 1 part glycerine, 0.2 parts polyethylene glycol 200, and 0.1 part potassium citrate glycerol.
0.12 parts food grade citric yellow.
0.3 parts edible lemon essences.

Soak the Hydroxypropyl pea starch in water in the gel tank at the temperature of 25 °C for 1.5 hours to from Liquid A. Mix carrageenan,pectin, glycerin, polyethylene glycol 2000, citric yellow, and potassium citrate in proper amount of water and stir for 15 minutes at 20 °C, and then heat the mix up to 60 °C to form liquid B. Add water to lemon essence at 15 °C and mix for 5 minutes to form liquid C, keep airtight and save for use. Heat up liquid A, and when the temperature reaches 65 °C, add liquid B into it and stir for 35 minutes, and then continue to heat up to 95 °C, staying at this temperature for 1.5 hour. Cool down, and when the temperature drops to 50 °C, add liquid C and well mix, and maintain this temperature for 1 hour. Stay static to defoam and then apply such made film-forming composite to make 1000 capsules by rotating die method.

### Embodiment 6

Hydroxypropyl corn starch: 5 parts; hydroxypropyl content is: 3.1%.
Water: 25 parts.

Appropriate adjuvant used to improve softgel capsule performance: 1.4 parts, which include 0.3 parts xanthan gum, 0.25 parts pectin, 0.6 parts glycerine, 0.1 parts polyethylene glycol 200, 0.1 parts polyethylene glycol 400, and 0.05 parts calcium citrate
0.1 parts edible citric yellow;
0.3 parts pineapple flavor essence

Soak the Hydroxypropyl corn starch in water in the gel tank at the temperature of 25 °C for 2 hours to from Liquid A. Mix xanthan gum, pectin, glycerol, polyethylene glycol 200, polyethylene glycol 400, edible citric yellow, and calcium citrate in proper amount of water for 15 minutes at 25 °C, and then heat the mix up to 55 °C to form liquid B. Add water to edible pineapple flavor essence at 20°C and mix for 10 minutes to form liquid C, keep airtight and save for use. Heat up liquid A, and when the temperature reaches 55 °C, add liquid B into it and stir for 30 minutes, and then continue to heat up to 98 °C, staying at this temperature for 1.5 hour. Cool down, and when the temperature drops to 50 °C, add liquid C and well mix, and maintain this temperature for 1 hour. Stay static to defoam and then apply such made film-forming composite to make 1000 capsules by rotating die method.

### Embodiment 7

Hydroxypropyl rice starch: 5 parts; hydroxypropyl content: 2.1%
Water: 15 parts

Appropriate adjuvant used to improve softgel capsule performance: 0.5 parts, which include 0.15 parts hydroxypropyl methyl cellulose, 0.1 parts seaweed polysaccharides, 0.24 parts polyethylene glycol 200, and 0.01 parts potassium Chloride
0.05 parts Edetate calcium
0.11 parts food grade carmine;
0.1 parts edible grade apple essence.

Soak the Hydroxypropyl rice starch in water in the gel tank at the temperature of 25 °C for 2 hours to from Liquid A. Mix hydroxypropyl methyl cellulose, polyethylene glycol 200, edible seaweed polysaccharides, and carmine in proper amount of water for 5 minutes at 25 °C, and then heat the mix up to 65 °C to form liquid B. Add water to edible apple flavor essence at 20 °C and mix for 10 minutes to form liquid C, keep airtight and save for use. Heat up liquid A, and when the temperature reaches 65 °C, add liquid B into it and stir for 20 minutes, and then continue to heat up to 98 °C, staying at this temperature for 2 hours. Cool down, and when the temperature drops to 50 °C, add liquid C and well mix, and maintain this temperature for 1.5 hours. Stay static to defoam and then apply such made film-forming composite to make 1000 capsules by rotating die method.

### Contrasting Example 1

hydroxypropyl tapioca starch: 5 parts; hydroxypropyl content: 6.1%.
Water: 40 parts.

Appropriate adjuvant used to improve softgel capsule performance: 1.4 parts, which include 0.7 part carrageenan and 0.7 part glycerol

Soak the hydroxypropyl tapioca starch in water in the gel tank at the temperature of 25 °C for 1.5 hours to from Liquid A. Mix carrageenan and glycerol in proper amount of water for 10 minutes at 20 °C, and then heat the mix up to 60 °C to form liquid B. Heat up to 95 °C and add liquid B and maintain the temperature for 1.5 hours. Cool down, and when the temperature drops to 50 °C, maintain this temperature for 1 hour. Stay static to defoam and then apply such made film-forming composite to make 1000 capsules by rotating die method.

### contrasting example 2

hydroxypropyl tapioca starch: 5 parts; with 6.1%.hydroxypropyl content.
Water: 40 parts.

Appropriate adjuvant used to improve softgel capsule performance: 4 parts, which include 2 parts carrageenan and 2 parts glycerol.

Soak the hydroxypropyl tapioca starch in water in the gel tank at the temperature of 25 °C for 1.5 hours to from Liquid A. Mix carrageenan and glycerol in proper amount of water for 10 minutes at 20 °C, and then heat the mix up to 60 °C to form liquid B. Heat up liquid A to 95 °C, add liquid B and maintain the temperature for 1.5 hours. Cool down, and when the temperature drops to 50 °C, maintain this temperature for 1 hour. Stay static to defoam and then apply such made film-forming composite to make 1000 capsules by rotating die method.

### Contrasting example 3

hydroxypropyl tapioca starch: 5 parts; hydroxypropyl content: 6.1%
Water: 40 parts.

Soak the Hydroxypropyl tapioca starch in water in the gel tank at the temperature of 25 °C for 1.5 hours to from Liquid A. Heat up liquid A to 95 °C, add liquid B and maintain the temperature for 1.5 hours. Cool down, and when the temperature drops to 50 °C, maintain this temperature for 1 hour. Stay static to defoam and then apply such made film-forming composite to make 1000 capsules by rotating die method.

### Contrasting example 4

Tapioca starch: 5 parts;
Water: 40 parts.

Appropriate adjuvant used to improve softgel capsule performance: 4 parts, which include 2 parts carrageenan and 2 parts glycerol.

Soak the Tapioca starch in water in the gel tank at the temperature of 25 °C for 1 hours to from Liquid A. Mix carrageenan and glycerol in proper amount of water for 10 minutes at 20 °C, and then heat the mix up to 60 °C to form liquid B. Heat up liquid A, and when the temperature reaches 60 °C, add liquid B into it and stir for 30 minutes, and then continue to heat up to 95 °C, maintaining this temperature for 1.5 hour. Cool down, and when the temperature drops to 50 °C, add liquid C and well mix, and maintain this temperature for 1 hour. Stay static to defoam and then apply such made film-forming composite to make 1000 capsules by rotating die method.

### Contrast example 5

Phosphate esterified tapioca starch: 5 parts.
Water: 40 parts.

Appropriate adjuvant used to improve softgel capsule performance: 1.4 parts, which include 0.7 parts carrageenan and 0.7 parts glycerol.

Soak the in water in the gel tank at the temperature of 25°C for 1 hours to from Liquid A. Mix carrageenan and glycerol in proper amount of water for 10 minutes at 20 °C, and then heat the mix up to 60 °C to form liquid B. Heat up liquid A, and when the temperature reaches 60 °C, add liquid B into it and stir for 30 minutes, and then continue to heat up to 95 °C, staying at this temperature for 1.5 hour. Cool down, and when the temperature drops to 50 °C, maintain this temperature for 1 hour. Stay static to defoam and then apply such made film-forming composite to make 1000 capsules by rotating die method.

### Contrast example 6

Phosphate esterified tapioca starch: 5 parts
Water: 40 parts

Appropriate adjuvant used to improve softgel capsule performance: 4 parts, which include 2 parts carrageenan and 2 parts glycerol.

Soak the in water in the gel tank at the temperature of 25 °C for 1.5 hours to from Liquid A. Mix carrageenan and glycerol in proper amount of water for 10 minutes at 20 °C, and then heat the mix up to 60 °C to form liquid B. Heat up liquid A to 95 °C, add liquid B and maintain the temperature for 1.5 hours, Cool down, and when the temperature drops to 50 °C, maintain this temperature for 1 hour. Stay static to defoam and then apply such made film-forming composite to make 1000 capsules by rotating die method.

### Contrasting example 7

1000 softgel capsules prepared according embodiment 1 in the Patent CN103228269A

### Contrasting example 8

1000 softgel capsules prepared according embodiment 4 in the Patent CN103495176A

### Contrasting example 10

1000 softgel capsules prepared according to embodiment 2 in the Patent CN103550065A

Embodiment 8: Determine the friability, and disintegration time of the capsules prepared by the methods of above embodiments and contrasting examples. Refer to the attached table 1 for the results.

FRIABILITY: Test Method: Put 50 capsules on a watch-glass and place it in the desiccator filled with saturated solution of magnesium nitrate for 24 hours under constant 25 ± 1 °C; Then take out the capsules and immediately place them inside a glass tube (diameter: 24mm, length: 200mm) upright on a 2cm thick wooden board. Allow a cylindrical weight (made of polytetrafluoroethylene, diameter: 22mm, weight: 20g ± 0.1g) to fall freely from the top surface level of the glass .tube. Acceptance criterion: No rupture is allowed, or if there is rupture, it should be limited to less than 5 capsules.

DISINTEGRATION TIME: Use 6 capsules as a sample and apply the Disintegration Time Determination Method for Capsules (Refer to Appendix X A in Chinese Pharmacopeia) to examine the disintegration time. Acceptance criterion: All the sample capsules tested must be completely disintegrated or dissolved within 30 minutes, if not, repeat the test using a new sample of 6 capsules and the result must meet the criterion.

Table 1 : Tightness and disintegration time of the capsules prepared by the methods in embodiment 1∼7 and the contrast example 1∼9.

| SN# | friability (number of capsules) | disintegration time (min) |
|---|---|---|
| Embodiment 1 | 0 | 10 |
| Embodiment 2 | 0 | 11 |
| Embodiment 3 | 0 | 10 |
| Embodiment 4 | 0 | 8 |
| Embodiment 5 | 0 | 8 |
| Embodiment 6 | 0 | 11 |
| Embodiment 7 | 0 | 10 |
| Constrasting Example 1 | 11 | 3 |
| Constrasting Example 2 | 10 | 22 |
| Constrasting Example 3 | 11 | Non-confirm |
| Constrasting Example 4 | 7 | Non-confirm |
| Constrasting Example 5 | 2 | 18 |
| Constrasting Example 6 | 4 | Non-confirm |
| Constrasting Example 7 | 2 | 17 |
| Constrasting Example 8 | 3 | 35 |
| Constrasting Example 9 | 2 | 32 |

As shown in table 1, the softgel capsules prepared using the technical proposal of this invention has excellent physical strength. The capsules prepared by the methods in embodiment of 1-7 have better friability and disintegration rate than those in contrasting examples 1∼9. This is one of the advantages of the softgel capsules described in this invention.

An embodiment provides a film-forming composite used to make starch softgel capsules and the method to prepare it. The said composite is made from water and hydroxypropyl starch by specific percentage, plus (optional) light-screening agent, colorant, and flavoring agent. Mix up the above components in sequential order and then process the mix into film-forming composite through controlled heating. The composite such made can be directly used to make starch softgel capsules by the conventional method applied for the gelatin capsule production.

## Claims

1. A film-forming composite comprising:
Hydroxypropyl starch at 20-95%;
Appropriate adjuvant used to improve softgel capsule performance at 0-25%;
Water at 5%∼80%;
Light-screening agent at 0∼2%;
Colorant at 0-2%; and
Flavoring agent at 0-2%,
wherein the adjuvant is one or a combination of a gelling agent, softening agent, and film forming agent by the percentage of 0-55% (GA), 0-65%(SA), and 0-5%(FFA) respectively;
said gelling agent is natural plant polysaccharides or microbial extracellular polysaccharide;
said softening agent is multiple fatty alcohol or aliphatic polyol polymer; and
said film-forming agent is selected from soluble calcium salt or potassium salt.

2. The composite of claim 1, wherein
the gelling agent is one or a combination of the substances selected from the group consisting of carrageenan, xanthan gum, guar gum, seaweed polysaccharides, pectin, hydroxypropyl methyl cellulose, and carboxymethyl cellulose;
said softening agent is one or a combination the substances selected from the group consisting of glycerol, polyethylene glycol 200, and polyethylene glycol 400; and
said film-forming agent is one or a combination of the substances selected from the group consisting of calcium chloride, potassium chloride, calcium citrate, and potassium citrate.

3. The composite according to claim 1 or 2, wherein said hydroxypropyl starch is one or a combination of hydroxypropylated starches selected from the group consisting of hydroxypropylated starches derived from mung beans, wheat, potato, cassava, soybean, with 2.0∼2.7% hydroxypropyl content.

4. The composite according to claim 1, 2 or 3, wherein
light-screening agent is calcium edentate or titanium dioxide or vanillin, and the colorant is of food or pharmaceutical grade; and
the flavoring agent is one or a combination of edible essence selected from the group consisting of the essence of different flavors such as apple, mint, lemon, banana, and pineapple.

5. A starch-based softgel capsule made from the combination of any type substance from claim 1, 2, 3 or 4, through the dropping-pill or rotary-die method.

6. A preparation method of a film-forming composite used to make starch softgel capsules, comprising:
Hydroxypropyl starch at 5 parts;
Water at 5∼40 parts;
Adjuvant used to improve softgel capsule performance at 0∼2 parts;
Light-screening agent at 0-0.3 parts;
Colorant at 0-0.5 parts;
Flavoring agent at 0-0.3 parts;
wherein the adjuvant used to improve softgel capsule performance is one or a combination of the substances selected from the group consisting of gelling agent, softening agent, and film forming agent, by the percentage of 0-55%(gelling agent), 0∼65% (softening agent), 0∼5%( film-forming agent) respectively;
wherein the gelling agent is made from natural plant polysaccharides or microbial extracellular polysaccharide;
wherein the softening agent is multiple fatty alcohol or aliphatic polyol polymer;
wherein the film-forming agent is selected from soluble calcium salt or potassium salt;
wherein the process comprises:
(1) Preparation of liquid A comprising allow hydroxypropyl starch to be soaked in water in the gel tank for 0.5∼2 hours at 20-25°C to form liquid A;
(2) Preparation of liquid B comprising mix the adjuvant used to improve softgel capsule performance, light-screening agent, and colorant in water; stir for 5-10minuts; and then heat up to 55-65°C within 10 minutes to form liquid B;
(3) Preparation of liquid C comprising mix water and flavoring agent for 5-10 minutes at the temperature of 15-20°C to form liquid C;
(4) Heat up the liquid A to 80-100°C, during which, liquid B can be added in as an option; mix well and temperature-preservation stir for 1-2 hours to form feed-liquid; and
(5) optionally, add liquid C into liquid A or Liquid D when the temperature falls to 50-60°C; stir and uniformly mix; maintain the temperature for 0.5∼1.5 hours; and keep airtight and save for use.

7. The method according to claim 6, wherein 20-35 minute temperature-preservation stirring is required at the point of 55-65°C in the middle of the process of heating up to 80-100 °C before continuing to raise the temperature.

8. The method according to claim 6 or 7, wherein
said gelling agent is one or a combination of the substances selected from the group consisting of carrageenan, xanthan gum, guar gum, seaweed polysaccharides, pectin, hydroxypropyl methyl cellulose, and carboxymethyl cellulose, and;
the softening agent is one or a combination of the substances selected from the group consisting of glycerol, polyethylene glycol 200, and polyethylene glycol 400; and,
the film-forming agent is one or a combination of the substances selected from the group consisting of calcium chloride, potassium chloride, calcium citrate, and potassium citrate.

9. The method according to claim 6, 7 or 8, wherein said hydroxypropyl starch is one or a combination of hydroxypropylated starches selected from the group consisting of hydroxypropylated starches derived from mung beans, wheat, potato, cassava, soybean, and corn, with 2.0∼7.0% hydroxypropyl content.

10. The method according to claim 6, 7, 8 or 9, wherein said light-screening agent is calcium edetate, or titanium dioxide, or vanillin, said colorant is of food or pharmaceutical grade; and
said flavoring agent is edible essence selected from the group consisting of the essence of different flavors such as apple, mint, lemon, banana and pineapple.
